(19) Europäisches Patentamt
European Patent Office
Office européen des brevets

(11) **EP 2 775 287 B1**

(12) **EUROPEAN PATENT SPECIFICATION**

(45) Date of publication and mention
of the grant of the patent:
**26.02.2020 Bulletin 2020/09**

(51) Int Cl.:
**G01N 21/59** *(2006.01)*  **G01N 21/85** *(2006.01)*
**G01N 33/28** *(2006.01)*  **G01N 21/25** *(2006.01)*

(21) Application number: **12845966.6**

(22) Date of filing: **01.11.2012**

(86) International application number:
**PCT/JP2012/078323**

(87) International publication number:
**WO 2013/065783 (10.05.2013 Gazette 2013/19)**

(54) **LUBRICANT OIL DEGRADATION SENSOR, SPEED REDUCER FOR INDUSTRIAL ROBOT, AND INDUSTRIAL ROBOT**

SCHMIERÖL-ZERSETZUNGSSENSOR, DREHZAHLMINDERER FÜR EINEN INDUSTRIEROBOTER UND INDUSTRIEROBOTER

CAPTEUR DE DÉGRADATION D'UNE HUILE LUBRIFIANTE, RÉDUCTEUR DE VITESSE POUR ROBOT INDUSTRIEL ET ROBOT INDUSTRIEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.11.2011 JP 2011242852**

(43) Date of publication of application:
**10.09.2014 Bulletin 2014/37**

(73) Proprietor: **Nabtesco Corporation
Tokyo 102-0093 (JP)**

(72) Inventor: **SHIMADA Hideshi
Tsu-shi, Mie 514-8533 (JP)**

(74) Representative: **Grünecker Patent- und Rechtsanwälte
PartG mbB
Leopoldstraße 4
80802 München (DE)**

(56) References cited:
**EP-A1- 2 647 979       WO-A2-03/030621
JP-A- H01 119 741     JP-A- H06 281 575
JP-A- H07 146 233     JP-A- H08 126 369
JP-A- S63 184 040     JP-A- 2005 156 297
JP-A- 2007 198 767    JP-A- 2007 278 903
JP-A- 2007 303 926    JP-A- 2008 128 933
JP-U- S62 192 242      US-A- 5 296 843
US-A1- 2008 024 761   US-A1- 2009 216 464**

**Description**

TECHNICAL FIELD

The present invention relates to a lubricant deterioration sensor for detecting the deterioration of lubricant of a machine.

BACKGROUND ART

Conventionally, as a lubricant deterioration sensor, an oil deterioration degree sensor has been known in which an oil entering gap portion for entering lubricant therein is formed on an optical path from an infrared LED (Light Emitting Diode) to a photo diode, and an amount of light absorption by the lubricant within the oil entering gap portion with respect to light emitted from the infrared LED is measured according to a light reception amount of the photo diode, to thereby determine a deterioration degree of the lubricant related to the light absorption amount thus measured (see patent literatures 1 and 2, for example).

However, the oil deterioration degree sensor described in each of the patent literatures 1 and 2 has a problem that, although a density of the insoluble content within the lubricant can be measured as the deterioration degree of the lubricant, kinds of contaminant within the lubricant can not be specified.

As a technique of specifying the kinds of contaminant within lubricant, a technique has been known in which an LED irradiates light toward a membrane filter having been used to filter the lubricant, and a light reception element converts reflection light from the contaminant on the membrane filter into digital values of RGB, to thereby specify the kinds of contaminant within the lubricant based on the RGB digital values thus converted (see non-patent literatures 1 and 2, for example).

Each of US 5 296 843 A, US 2009/216464 A1 and US 2008/024761 A1 discloses a lubricant deterioration sensor according to the preamble of claim 1.

EP 2 647 979 A1 is prior art under Article 54(3) EPC and also discloses a lubricant deterioration sensor according to the preamble of claim 1.

PRIOR ART LITERATURE

PATENT LITERATURE

Patent Literature 1: JP-A-7-146233
Patent Literature 2: JP-A-10-104160

NON-PATENT LITERATURE

Non-Patent Literature 1: Tomohiko Yamaguchi and four others, "METHOD OF DISCRIMINATING HUE OF CONTAMINANT WITHIN LUBRICANT", Fukui University, Faculty of Engineering, Research Report, March 2003, Volume 51, No. 1, pp. 81 - 88
Non-Patent Literature 2: Tomonori Honda, "DETERIORATION DIAGNOSIS OF LUBRICANT INSPECTION TECHNOLOGY", Journal of Japan Society for Precision Engineering, 2009, Volume 75, No. 3, pp. 359 - 362

SUMMARY OF INVENTION

TECHNICAL PROBLEM

However, since the technique described in each of the non-patent literatures 1 and 2 requires to drain lubricant from a machine and filter the lubricant by a membrane filter, there arises a problem of lacking immediacy.

Accordingly, an object of this invention is to provide a lubricant deterioration sensor which can immediately specify the kinds and amounts of contaminant within lubricant of a machine.

SOLUTION TO PROBLEMS

The present invention is defined in claim 1.

According to this invention, there is provided with a lubricant deterioration sensor to be fixed to a machine to detect deterioration of a lubricant in a lubricant-containing space of the machine, the lubricant deterioration sensor comprising:

> a light emitting element configured to emit light;
> a color light reception element configured to receive the light emitted from the light emitting element and detect color of the received light;
> a gap forming member forming an oil gap being a gap in which the lubricant enters;
> a supporting member supporting the light emitting element, the color light reception element and the gap forming member; and
> a fixing member to be fixed to the machine, wherein the gap forming member is disposed such that, when the lubricant deterioration sensor is fixed to the machine, the gap forming member is in the lubricant-containing space of the machine such that lubricant enters the oil gap,
> the gap forming member transmits the light emitted from the light emitting element, and
> the oil gap is disposed on an optical path from the light emitting element to the color light reception element,
> characterized in that the fixing member supports the supporting member rotatably so that a direction of an opening of the oil gap changes when the supporting member rotates relative to the fixing member.

The supporting member may include a rotational driving force receiving portion which is a portion for receiving a rotational driving force with respect to the supporting member from the outside by a contact force, at a position where the rotational driving force receiving portion does not contact with the lubricant in a state where the fixing member is fixed to the machine.

The lubricant deterioration sensor may include a rotation preventing member preventing a rotation of the supporting member with respect to the fixing member by contacting with both the supporting member and the fixing member, and

the rotation preventing member may include a contact driving force receiving portion which is a portion for receiving a driving force for contacting with both the supporting member and the fixing member from the outside by a contact force, at a position where the contact driving force receiving portion does not contact with the lubricant in a state where the fixing member is fixed to the machine.

The light emitting element may be a white LED which emits white light.

The gap forming member may have a reflection surface for bending the optical path.

The gap forming member may include two right-angle prisms each provided with the reflection surface for bending the optical path by 90 degrees, the optical path may be bent by 180 degrees by the reflection surfaces of the two right-angle prisms, and the oil gap may be formed between the two right-angle prisms.

The supporting member may have an optical path surrounding portion which surrounds at least a part of the optical path, and the optical path surrounding portion may have a surface which is treated to a treatment for preventing light reflection.

A surface of the gap forming member forming the oil gap may be treated by an oil repelling treatment.

According to this invention, there is provided with a reduction gear for an industrial robot which includes the lubricant deterioration sensor and a reduction gear body as the machine.

According to this invention, there is provided with an industrial robot which includes the lubricant deterioration sensor and an industrial robot body as the machine, wherein the industrial robot body includes an arm and a reduction gear used at an articular portion of the arm, and the lubricant deterioration sensor is a sensor for detecting deterioration of lubricant of the reduction gear.

ADVANTAGEOUS EFFECTS OF INVENTION

In the lubricant deterioration sensor according to this invention, since the color light reception element detects colors with respect to the light having wavelengths not absorbed by the contaminant such as ion powder within the lubricant at the oil gap among the light emitted by the light emitting element, colors of the contaminant within the lubricant of the machine can be detected immediately. That is, the lubricant deterioration sensor according to this invention can immediately specify the kinds and amounts of the contaminant within the lubricant of the machine based on the colors detected by the color light reception element. Further, in the lubricant deterioration sensor according to this invention, in a case where the fixing member is fixed to the machine, the direction of the opening of the oil gap in a state where the fixing member is fixed to the machine can be adjusted so that the detection accuracy of the deterioration of the lubricant of the machine becomes high.

In the lubricant deterioration sensor according to this invention, after the fixing member is fixed to the machine, the direction of the opening of the oil gap in the state where the fixing member is fixed to the machine can be adjusted so that the detection accuracy of the deterioration of the lubricant of the machine becomes high.

In the lubricant deterioration sensor according to this invention, after the fixing member is fixed to the machine, the direction of the opening of the oil gap at the time of fixing the fixing member to the machine can be fixed so that the detection accuracy of the deterioration of the lubricant of the machine becomes high.

The lubricant deterioration sensor according to this invention can be miniaturized as compared with a configuration where the light emitting element is a lamp other than an LED, for example.

In the lubricant deterioration sensor according to this invention, as compared with a configuration where the optical path from the light emitting element to the color light reception element is straight, the entire configuration can be miniaturized by disposing the light emitting element and the color light reception element close to each other. Further, since the gap forming member has a function of bending the optical path as well as a function of forming the oil gap, the number of the components can be reduced as compared with a configuration where a member for bending the optical path is separately provided in place of the gap forming member.

The lubricant deterioration sensor according to this invention can be miniaturized with a simple configuration having a small number of the components.

In the lubricant deterioration sensor according to this invention, since the color light reception element is prevented from receiving unnecessary reflection light, the detection accuracy of the colors of the contaminant within the lubricant can be improved as compared with a configuration where the color light reception element receives unnecessary reflection light.

Since the lubricant deterioration sensor according to this invention is configured to easily flow the lubricant through the oil gap, the detection accuracy of the colors of the contaminant within the lubricant can be improved as compared with a configuration where the lubricant likely remains at the oil gap. Further, in the lubricant deterioration sensor according to this invention, when the surface forming the oil gap is treated to the oil repelling treatment, since dirt is unlikely adhered to the surface forming the oil gap, the degradation of the detection accuracy of the colors of the contaminant within the lubricant due to the adhesion of dirt can be suppressed.

The lubricant deterioration sensor of the reducer for an industrial robot can immediately specify the kinds and amounts of the contaminant within the lubricant of the reducer body based on the colors detected by the color light reception element. Thus, the reducer for an industrial robot according to this invention can immediately predict a failure. Further, in the lubricant deterioration sensor of the reducer for an industrial robot, the direction of the opening of the oil gap at the time of fixing the fixing member to the machine can be adjusted. Thus, the reducer for an industrial robot according to this invention can predict a failure with a high accuracy.

The lubricant deterioration sensor for an industrial robot according to this invention can immediately specify the kinds and amounts of the contaminant within the lubricant of the reducer based on the colors detected by the color light reception element. Thus, the industrial robot according to this invention can immediately predict a failure. Further, in the lubricant deterioration sensor for the industrial robot, the direction of the opening of the oil gap at the time of fixing the fixing member to the machine can be adjusted. Thus, the industrial robot according to this invention can predict a failure with a high accuracy.

The lubricant deterioration sensor according to this invention can immediately specify the kinds and amounts of the contaminant within the lubricant of the machine.

BRIEF DESCRIPTION OF THE DRAWINGS

[Fig. 1] Fig. 1 is a side view of an industrial robot according to an embodiment of this invention.
[Fig. 2] Fig. 2 is a sectional view of the articular portion of the industrial robot shown in Fig. 1.
[Fig. 3] Fig. 3 is a front view of a lubricant deterioration sensor shown in Fig. 2.
[Fig. 4] Fig. 4 is a front sectional view of the lubricant deterioration sensor shown in Fig. 3 in an attached state to an arm.
[Fig. 5] Fig. 5(a) is a plan view of the lubricant deterioration sensor shown in Fig. 3. Fig. 5(b) is a bottom view of the lubricant deterioration sensor shown in Fig. 3.
[Fig. 6] Fig. 6(a) is a front view of a housing shown in Fig. 3. Fig. 6(b) is a front sectional view of the housing shown in Fig. 3.
[Fig. 7] Fig. 7(a) is a side view of the housing shown in Fig. 3. Fig. 7(b) is a side sectional view of the housing shown in Fig. 3.
[Fig. 8] Fig. 8(a) is a plan view of the housing shown in Fig. 3. Fig. 8(b) is a bottom view of the housing shown in Fig. 3.
[Fig. 9] Fig. 9(a) is a front view of a holder shown in Fig. 3. Fig. 9(b) is a front sectional view of the holder shown in Fig. 3.

[Fig. 10] Fig. 10(a) is a side view of the holder shown in Fig. 3. Fig. 10(b) is a side sectional view of the holder shown in Fig. 3.

[Fig. 11] Fig. 11(a) is a plan view of the holder shown in Fig. 3. Fig. 11(b) is a bottom view of the holder shown in Fig. 3.

[Fig. 12] Fig. 12 is a diagram showing an optical path from a white LED to an RGB sensor shown in Fig. 4.

[Fig. 13] Fig. 13(a) is a front view of a holder cap shown in Fig. 4. Fig. 13(b) is a front sectional view of the holder cap shown in Fig. 4.

[Fig. 14] Fig. 14(a) is a plan view of the holder cap shown in Fig. 4. Fig. 14(b) is a bottom view of the holder cap shown in Fig. 4.

[Fig. 15] Fig. 15 is a diagram showing an example of the relation between the direction of the opening of an oil gap shown in Fig. 3 with respect to the flow of lubricant and a color difference ∆E of colors detected by the RGB sensor with respect to black.

[Fig. 16] Fig. 16(a) is a diagram showing a state that the direction of the opening of the oil gap shown in Fig. 3 with respect to the flow of the lubricant is 0 degree. Fig. 16(b) is a diagram showing a state that the direction of the opening of the oil gap shown in Fig. 3 with respect to the flow of the lubricant is 45 degrees. Fig. 16(c) is a diagram showing a state that the direction of the opening of the oil gap shown in Fig. 3 with respect to the flow of the lubricant is 90 degrees.

DESCRIPTION OF EMBODIMENTS

Hereinafter, an embodiment of this invention will be explained with reference to drawings.

First, the configuration of an industrial robot according to this embodiment will be explained.

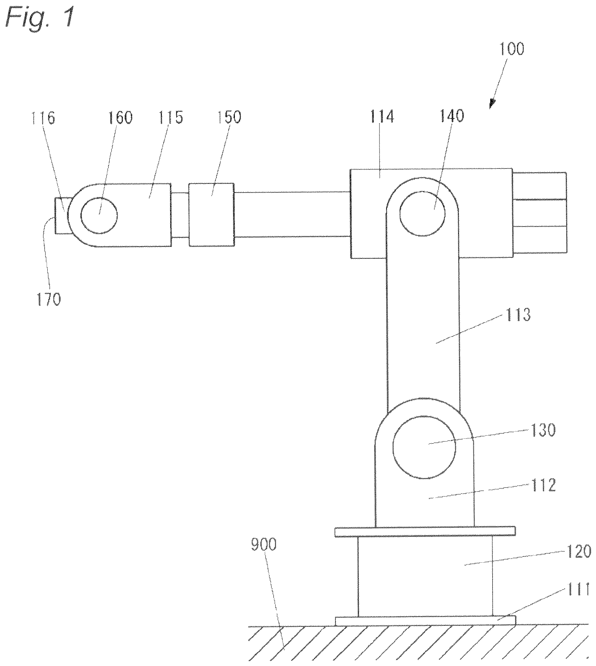

Fig. 1 is a side view of the industrial robot 100 according to this embodiment.

As shown in Fig. 1, the industrial robot 100 includes an attachment portion 111 to be attached to an installation portion 900 such as a floor or a ceiling, arms 112 to 116, an articular portion 120 for connecting between the attachment portion 111 and the arm 112, an articular portion 130 for connecting between the arm 112 and an arm 113, an articular portion 140 for connecting between the arm 113 and an arm 114, an articular portion 150 for connecting between the arm 114 and an arm 115, an articular portion 160 for connecting between the arm 115 and an arm 116, and an articular portion 170 for connecting between the arm 116 and a not-shown hand.

Of the industrial robot 100, portions except for the lubricant deterioration sensors such as lubricant deterioration sensors 137a, 137b, 139a, 139b described later constitute the industrial robot body according to this invention.

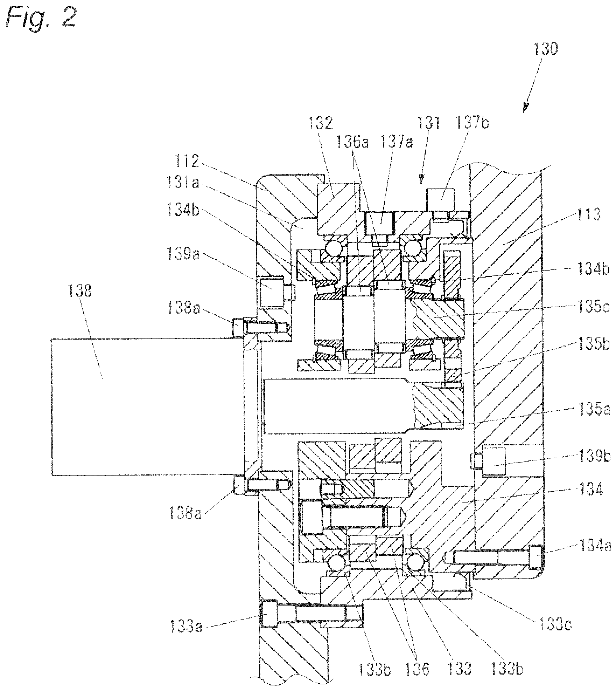

Fig. 2 is a sectional view of the articular portion 130. Although the explanation is made hereinafter as to the articular portion 130, the configuration of each of the articular portions 120, 140 to 170 is substantially same.

As shown in Fig. 2, the articular portion 130 includes a reducer 131 as a reducer for the industrial robot according to this invention for connecting between the arm 112 and the arm 113, a motor 138 fixed to the arm 112 by means of bolts 138a, and the lubricant deterioration sensors 139a, 139b each for detecting the deterioration of the lubricant 131a of the reducer 131.

The reducer 131 includes a reducer body 132 and the lubricant deterioration sensors 137a and 137b each for detecting the deterioration of the lubricant 131a of the reducer body 132.

The reducer body 132 includes a case 133 fixed to the arm 112 by means of bolts 133a, a supporting body 134 fixed to the arm 113 by means of bolts 134a, a gear 135a fixed to the output shaft of the motor 138, three gears 135b which are disposed around the center axis of the reducer 131 with a constant interval therebetween and mesh with the gear 135a, three crank shafts 135c which are disposed around the center axis of the reducer 131 with a constant interval therebetween and fixed to the gears 135b, respectively, and two external gears 136 which mesh with internal gears provided at the case 133.

The supporting body 134 is rotatably supported by the case 133 via bearings 133b. A seal member 133c for preventing leakage of the lubricant 131a is provided between the case 133 and the supporting body 134.

Each of the crank shafts 135c is rotatably supported by the supporting body 134 via bearings 134b and further rotatably supported by the external gears 136 via bearings 136a.

Each of the lubricant deterioration sensor 137a and the lubricant deterioration sensor 137b is fixed to the case 133. The lubricant deterioration sensor 139a is fixed to the arm 112. The lubricant deterioration sensor 139b is fixed to the arm 113.

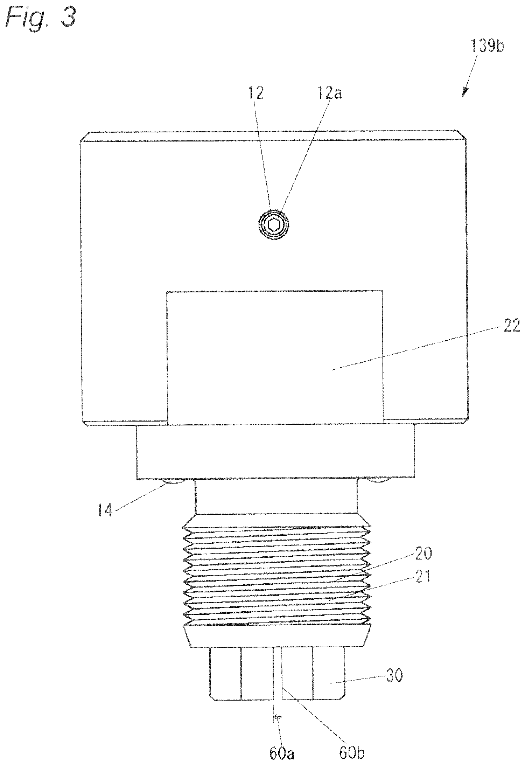

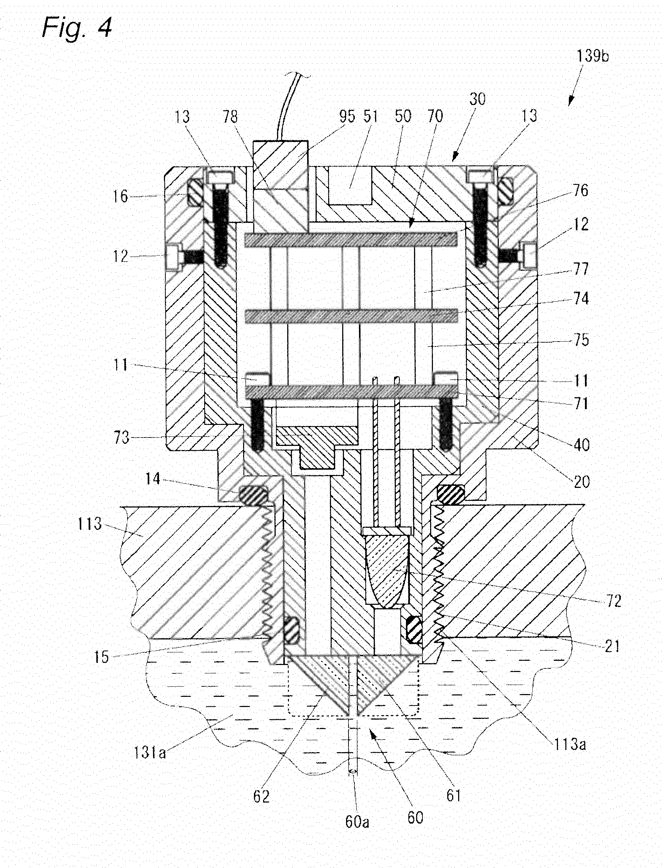

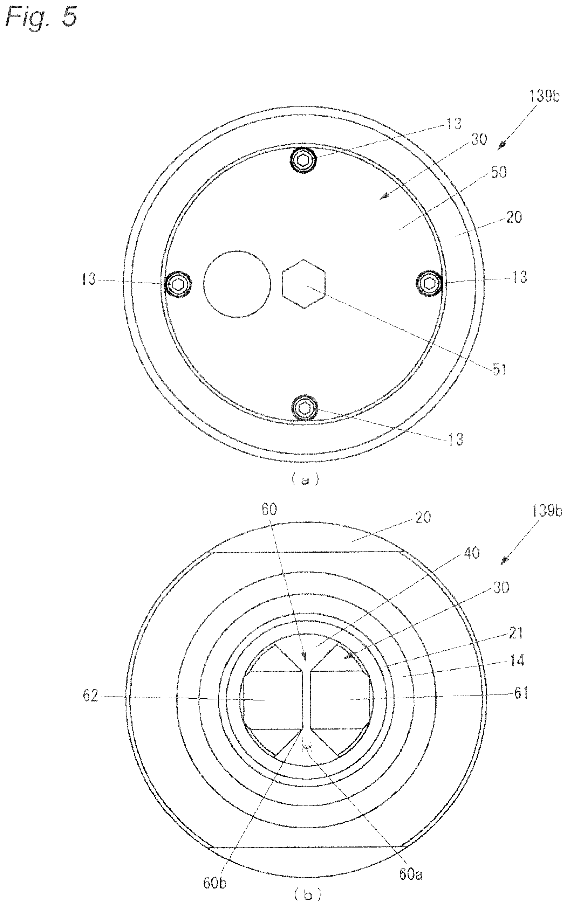

Fig. 3 is a front view of the lubricant deterioration sensor 139b. Fig. 4 is a front sectional view of the lubricant deterioration sensor 139b in an attached state to the arm 113. Fig. 5(a) is a plan view of the lubricant deterioration sensor 139b. Fig. 5(b) is a bottom view of the lubricant deterioration sensor 139b. Although the explanation is made hereinafter as to the lubricant deterioration sensor 139b, the configuration of each of the lubricant deterioration sensors such as the lubricant deterioration sensors 137a, 137b, 139a other than the lubricant deterioration sensor 139b is substantially same.

As shown in Figs. 3 to 5, the lubricant deterioration sensor 139b includes a housing 20 made of aluminum alloy for supporting the respective components of the lubricant deterioration sensor 139b, a supporting member 30 for supporting a white LED 72, an RGB sensor 73 and a gap forming member 60 described later, the gap forming member 60 held by the supporting member 30, and an electronic component group 70 having the white LED 72 and the RGB sensor 73.

The supporting member 30 is fixed to the housing 20 by means of bolts 12 with hexagon holes. The supporting member 30 includes a holder 40 made of aluminum alloy, and a holder cap 50 made of aluminum alloy which is fixed to the holder 40 by means of bolts 13 with hexagon holes.

The gap forming member 60 is constituted by two right-angle prisms 61, 62 made of glass. An oil gap 60a as a gap for entering the lubricant 131a therein is formed between the two right-angle prisms 61, 62.

The electronic component group 70 includes a circuit board 71 fixed to the supporting member 30 by means of screws 11, the white LED 72 mounted on the circuit board 71, the RGB sensor 73 mounted on the circuit board 71, a circuit board 74 disposed at one surface side of the circuit board 71 opposing to the white LED 72 and RGB sensor 73 side thereof, a plurality of pillars 75 for fixing the circuit board 71 and the circuit board 74, a circuit board 76 disposed on the opposite surface side of the circuit board 71 with respect to the circuit board 74, a plurality of pillars 77 for fixing the circuit board 74 and the circuit board 76, and a connector 78 mounted on one surface side of the circuit board 76 opposing to the circuit board 74 side thereof. A plurality of electronic components are mounted on each of the circuit board 71, the circuit board 74 and the circuit board 76. The circuit board 71, the circuit board 74 and the circuit board 76 are mutually connected electrically.

The lubricant deterioration sensor 139b includes an O ring 14 for preventing the leakage of the lubricant 131a from a gap between the housing 20 and the arm 113, an O ring 15 for preventing the leakage of the lubricant 131a from a gap between the housing 20 and the holder 40, and an O ring 16 disposed between the housing 20 and the holder cap 50.

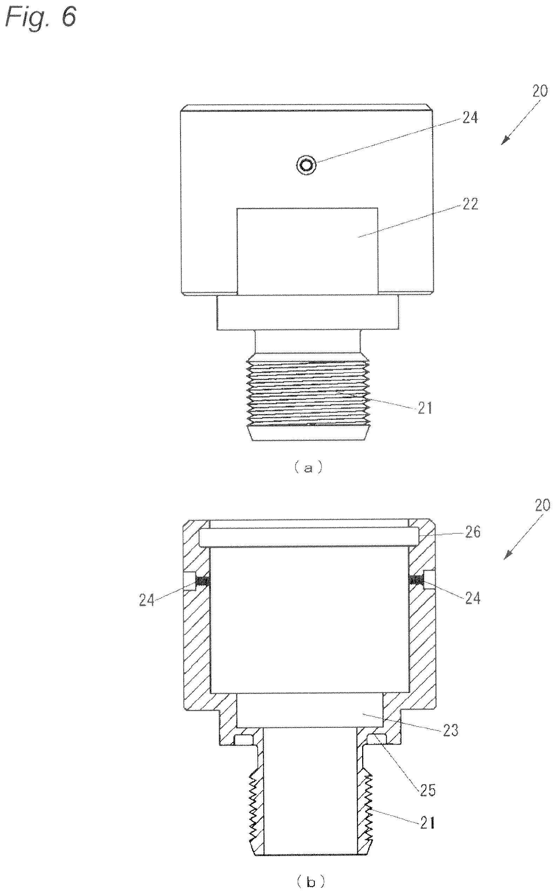

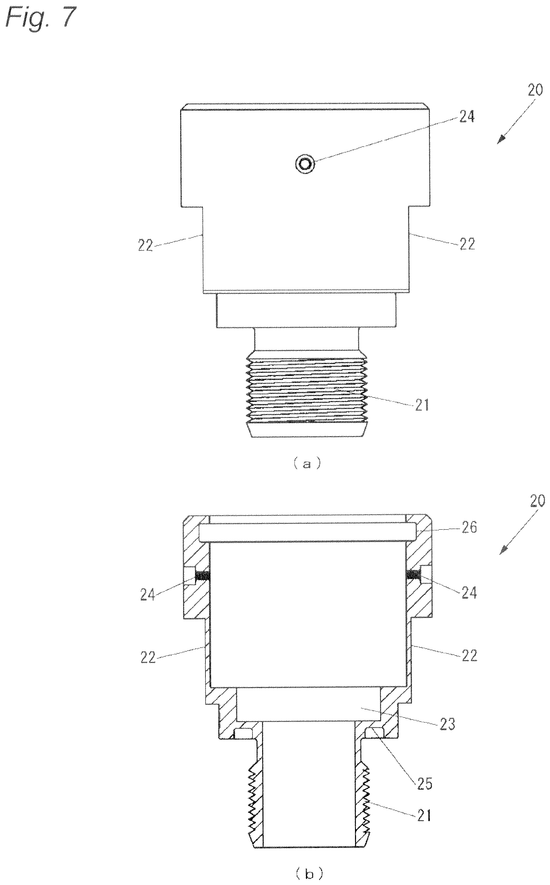

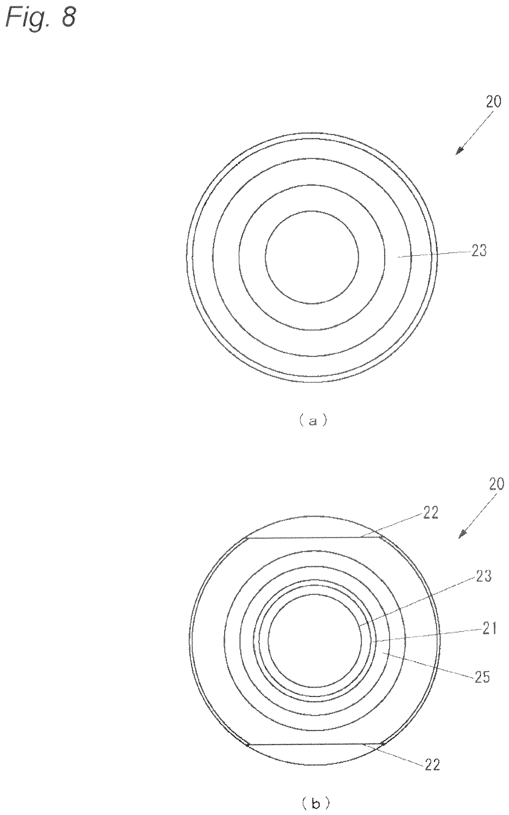

Fig. 6(a) is a front view of the housing 20. Fig. 6(b) is a front sectional view of the housing 20. Fig. 7(a) is a side view of the housing 20. Fig. 7(b) is a side sectional view of the housing 20. Fig. 8(a) is a plan view of the housing 20. Fig. 8(b) is a bottom view of the housing 20.

As shown in Figs. 3 to 8, the housing 20 includes a screw portion 21 to be fixed to the screw hole 113a of the arm 113, a tool contact portion 22 which is grasped by a tool such as a wrench at the time of rotating the screw portion 21 with respect to the screw hole 113a of the arm 113, and a holder housing portion 23 in which the holder 40 is housed. Further, the housing 20 is provided with screw holes 24 into which the bolts 12 with hexagon holes are screwed, a groove 25 into which the O ring 14 is fit, and a groove 26 into which the O ring 16 is fit. The housing 20 is configured to be fixed to the arm 113 of the industrial robot 100, that is, the industrial robot body and hence constitutes a fixing member of this invention.

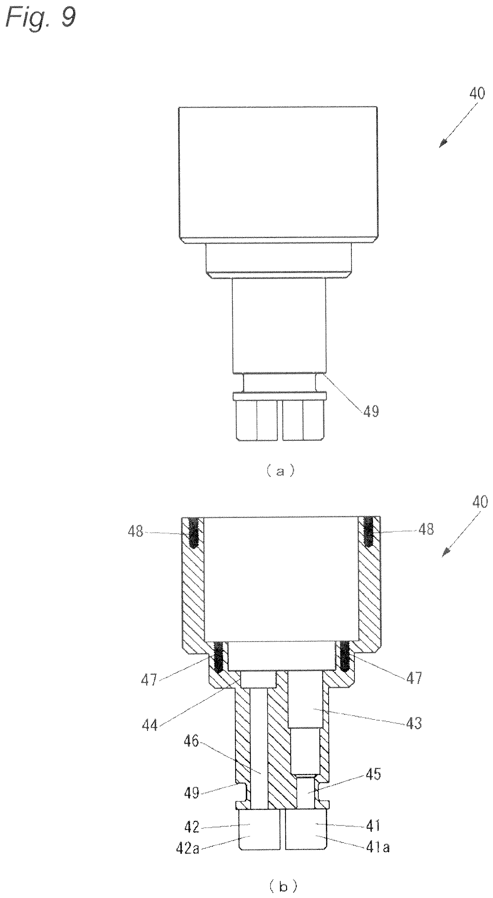

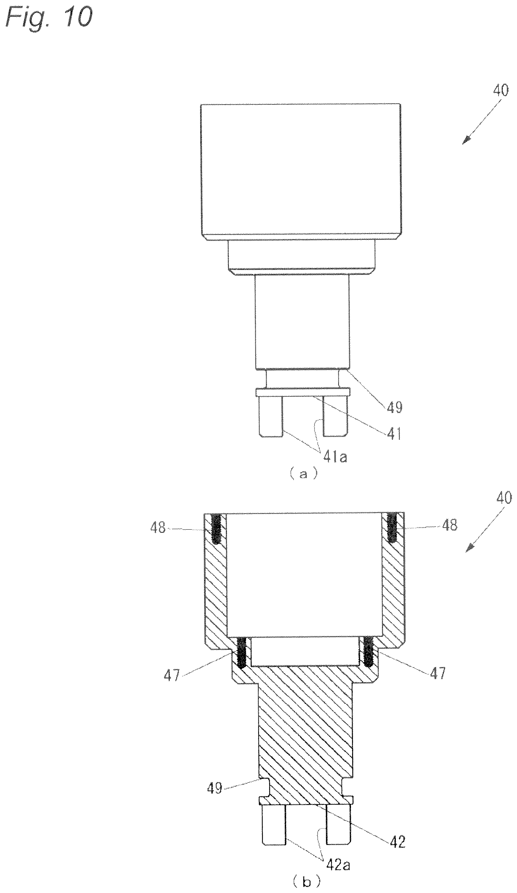

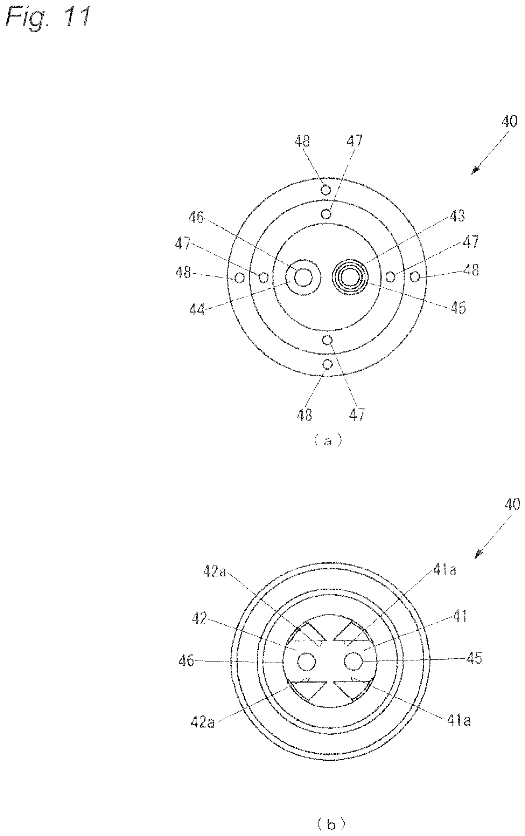

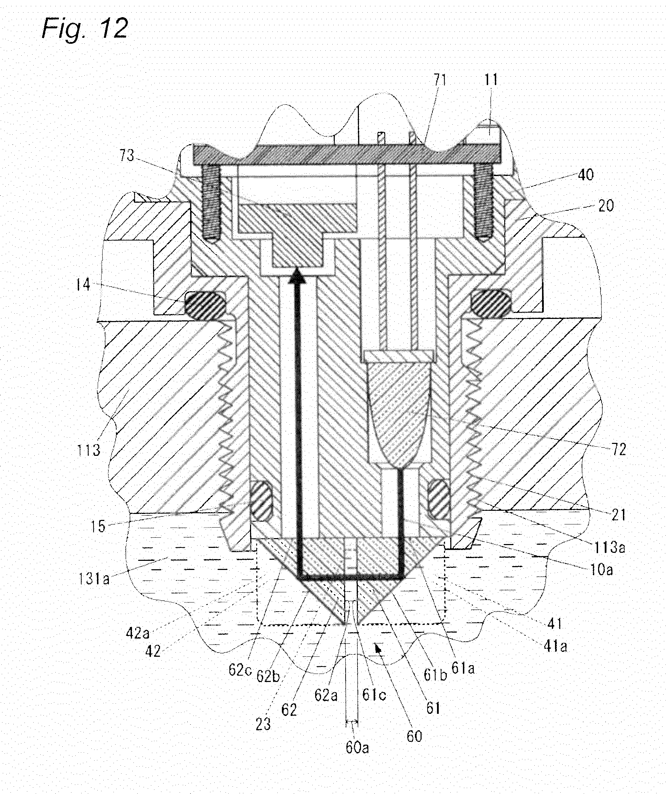

Fig. 9(a) is a front view of the holder 40. Fig. 9(b) is a front sectional view of the holder 40. Fig. 10(a) is a side view of the holder 40. Fig. 10(b) is a side sectional view of the holder 40. Fig. 11(a) is a plan view of the holder 40. Fig. 11(b) is a bottom view of the holder 40. Fig. 12 is a diagram showing an optical path 10a from the white LED 72 to the RGB sensor 73.

As shown in Figs.3 to 5 and Figs. 9 to 12, the holder 40 includes a prism housing portion 41 for housing the right-angle prism 61, a prism housing portion 42 for housing the right-angle prism 62, an LED housing portion 43 for housing the white LED 72, and an RGB sensor housing portion 44 for housing the RGB sensor 73. Further, the holder 40 is provided with a hole 45 penetrating the prism housing portion 41 and the LED housing portion 43, a hole 46 penetrating the prism housing portion 42 and the RGB sensor housing portion 44, screw holes 47 in which the screws 11 are threaded, screw holes 48 in which the bolts 13 with hexagon holes are threaded, and a groove 49 in which the O ring 15 is fitted.

The prism housing portion 41 includes two walls 41a sandwiching the right-angle prism 61 therebetween. The right-angle prism 61 is fixed to the walls 41a by means of adhesive. The prism housing portion 42 includes two walls 42a sandwiching the right-angle prism 62 therebetween. The right-angle prism 62 is fixed to the walls 42a by means of adhesive.

The holder 40 surrounds at least a part of the optical path 10a from the white LED 72 to the RGB sensor 73 by means of the LED housing portion 43, the hole 45, the prism housing portion 41, the prism housing portion 42, the hole 46 and the RGB sensor housing portion 44, thereby constituting an optical path surrounding portion according to this invention.

The surface of the holder 40 is treated to a treatment for preventing light reflection such as a black alumite treatment for matting.

The holder 40 supports the white LED 72 and the RGB sensor 73 via the circuit board 71. Further, the holder 40 directly supports the gap forming member 60.

As shown in Fig. 12, the oil gap 60a of the gap forming member 60 is disposed on the optical path 10a from the white LED 72 to the RGB sensor 73.

The right-angle prisms 61, 62 transmit light emitted from the white LED 72. The right-angle prism 61 is provided with a light incident surface 61a on which light emitted from the white LED 72 is made incident, a light reflection surface 61b which reflects the light entered from the light incident surface 61a in a manner of bending the propagation direction of the light by 90 degrees, and a light emission surface 61c which emits the light reflected by the light reflection surface 61b. The right-angle prism 62 is provided with a light incident surface 62a on which light emitted from the light emission surface 61c of the right-angle prism 61 is made incident, a light reflection surface 62b which reflects the light entered from the light incident surface 62a in a manner of bending the propagation direction of the light by 90 degrees, and a light emission surface 62c which emits the light reflected by the light reflection surface 62b.

Each of the light incident surface 61a, the light reflection surface 61b and the light emission surface 61c of the right-angle prism 61 and the light incident surface 62a, the light reflection surface 62b and the light emission surface 62c of the right-angle prism 62 is optically polished. Further, each of the light reflection surface 61b of the right-angle prism 61 and the light reflection surface 62b of the right-angle prism 62 is provided with an aluminum deposition film. Furthermore, an $SiO_2$ film is formed on the aluminum deposition film in order to protect the aluminum deposition film which is low in a degree of hardness and an adhesive force.

The optical path 10a is bent by 90 degrees by the light reflection surface 61b of the right-angle prism 61 and also bent by 90 degrees by the light reflection surface 62b of the right-angle prism 62. That is, the optical path 10a is bent by 180 degrees by the gap forming member 60.

When the length of the oil gap 60a is too short, since the contaminant within the lubricant 131a unlikely flows suitably through the gap 60a, the detection accuracy of the colors of the contaminant within the lubricant 131a degrades. On the other hand, when the length of the oil gap 60a is too long, the light emitted from the white LED 72 is excessively absorbed by the contaminant within the lubricant 131a within the oil gap 60a and hence unlikely reaches the RGB sensor 73. Thus, the detection accuracy of the colors of the contaminant within the lubricant 131a also degrades. Accordingly, preferably, the length of the oil gap 60a is set suitably so that the detection accuracy of the colors of the contaminant within the lubricant 131a becomes high. A distance between the light emission surface 61c of the right-angle prism 61 and the light incident surface 62a of the right-angle prism 62, that is, the length of the oil gap 60a is 1mm, for example.

The white LED 72 is an electronic component which emits white light and constitutes a light emitting element according to this invention. As the white LED 72, NSPW500GS-K1 manufactured by Nichia Corporation, for example, may be employed.

The RGB sensor 73 is an electronic component which detects the colors of the received light and constitutes a color light reception element. As the RGB sensor 73, S9032-02 manufactured by Hamamatsu Photonics K.K., for example, may be employed.

As shown in Fig. 4, the connector 78 is configured in a manner that the connector 95 of the external deice at the outside of the lubricant deterioration sensor 139b is connected thereto so as to be supplied with electric power via the connector 95 from the external deice, and the detection result of the lubricant deterioration sensor 139b is outputted as an electric signal to the external device via the connector 95.

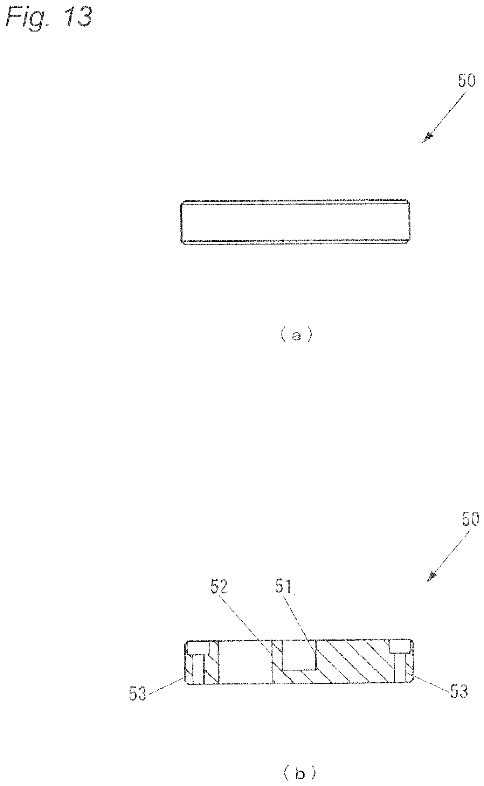

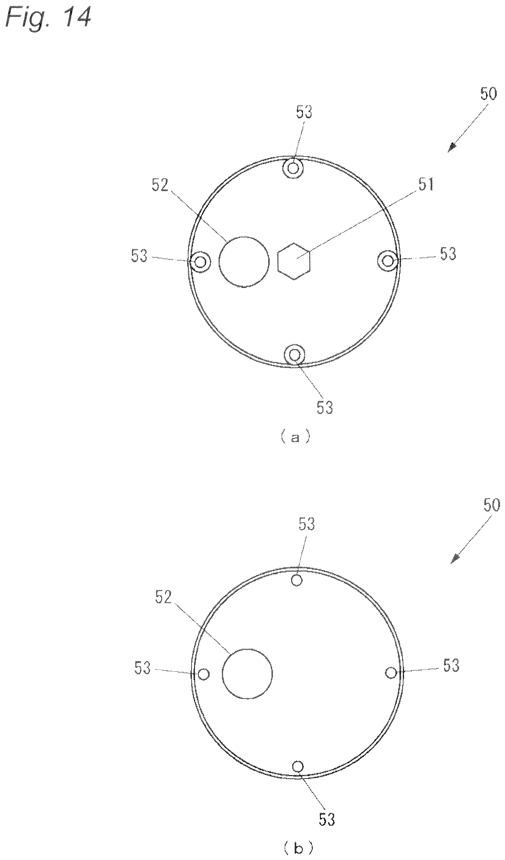

Fig. 13(a) is a front view of the holder cap 50. Fig. 13(b) is a front sectional view of the holder cap 50. Fig. 14(a) is a plan view of the holder cap 50. Fig. 14(b) is a bottom view of the holder cap 50.

As shown in Figs. 3 to 5 and Figs. 13 to 14, the holder cap 50 is provided with a tool contact portion 51 for contacting with a tool such as a hexagonal wrench at the time of rotating the supporting member 30 with respect to the housing 20. The tool contact portion 51 is a portion for receiving a rotational driving force of the supporting member 30 with respect to the housing 20 from the outside by a contact force, and constitutes a rotational driving force receiving portion according to this invention. The tool contact portion 51 is disposed at a position not contacting with the lubricant 131a when the housing 20 is fixed to the arm 113. Further, the holder cap 50 is provided with a hole 52 in which the connector 78 is inserted and holes 53 in which the bolts 13 with hexagon holes are inserted.

The surface of the holder cap 50 is treated to the treatment for preventing light reflection such as the black alumite treatment for matting.

As shown in Figs. 3 and 4, the bolts 12 with hexagon holes are configured so as to prevent the rotation of the supporting member 30 with respect to the housing 20 by contacting with both the supporting member 30 and the housing 20, and constitutes a rotation preventing member according to this invention. The bolt 12 with a hexagon hole has a tool contact portion 12a for contacting with a tool such as the hexagonal wrench. The tool contact portion 12a is a portion for receiving a driving force for contacting with both the supporting member 30 and the housing 20 from the outside by a contact force, and constitutes a contact driving force receiving portion according to this invention. The tool contact portion 12a is disposed at a position not contacting with the lubricant 131 a when the housing 20 is fixed to the arm 113.

Next, the assembling method of the lubricant deterioration sensor 139a will be explained. Hereinafter, although the explanation is made as to the lubricant deterioration sensor 139b, the method of each of the lubricant deterioration sensors such as the lubricant deterioration sensors 137a, 137b, 139a other than the lubricant deterioration sensor 139b is substantially same.

First, adhesive is pasted on the surface plane of the prism housing portion 41 of the holder 40 to be made in contact with the light incident surface 61a of the right-angle prism 61, and also adhesive is pasted on the two surface planes of the right-angle prism 61 to be made in contact with the two walls 41a of the prism housing portion 41. Then, the right-angle prism 61 is fixed to the prism housing portion 41 by the adhesive. Further, adhesive is pasted on the surface plane of the prism housing portion 42 of the holder 40 to be made in contact with the light emission surface 62c of the right-angle prism 62, and also adhesive is pasted on the two surface planes of the right-angle prism 62 to be made in contact with the two walls 42a of the prism housing portion 42. Then, the right-angle prism 62 is fixed to the prism housing portion 42 by the adhesive. Furthermore, the white LED 72 is fixed to the LED housing portion 43 of the holder 40 by means of adhesive.

Then, the circuit board 71 mounting the RGB sensor 73 thereon is fixed to the holder 40 by means of the screws 11, and the white LED 72 is fixed to the circuit board 71 by means of soldering. Further, various kinds of electronic components such as the connector 78 are assembled, thereby supporting the electronic component group 70 by the holder 40.

Next, the holder cap 50 is fixed to the holder 40 by means of the bolts 13 with hexagon holes.

Lastly, the holder 40 attached with the O ring 15 is fixed, by means of the bolts 12 with hexagon holes, to the holder housing portion 23 of the housing 20 attached with the O ring 14 and the O ring 16.

Next, the explanation will be made as to a method of mounting the lubricant deterioration sensor 139b to the arm 113. Hereinafter, although the explanation is made as to the lubricant deterioration sensor 139b, the method of each of the lubricant deterioration sensors such as the lubricant deterioration sensors 137a, 137b, 139a other than the lubricant deterioration sensor 139b is substantially same.

First, the tool contact portion 22 of the housing 20 is grasped by a tool and the screw portion 21 of the housing 20 is threaded into the screw hole 113a of the arm 113, thereby fixing the lubricant deterioration sensor 139b to the arm 113.

Then, the connector 95 of the external device at the outside of the lubricant deterioration sensor 139b is connected to the connector 78.

Next, the operation of the industrial robot 100 will be explained.

First, the operation of the articular portion 130 will be explained. Hereinafter, although the explanation is made as to the articular portion 130, the operation of each of the articular portions 120 and 140 to 170 is substantially same.

When the output shaft of the motor 138 of the articular portion 130 rotates, the rotation force of the motor 138 is reduced by the reducer 131, to thereby move the arm 113 fixed to the supporting body 134 of the recorder 131 with respect to the arm 112 fixed to the case 133 of the reducer 131.

Next, the operation of the lubricant deterioration sensor 139b will be explained. Hereinafter, although the explanation is made as to the lubricant deterioration sensor 139b, the operation of each of the lubricant deterioration sensors such as the lubricant deterioration sensors 137a, 137b, 139a other than the lubricant deterioration sensor 139b is substantially same.

The lubricant deterioration sensor 139b emits white light from the white LED 72 in response to electric power supplied from the external device via the connector 78.

Then, the lubricant deterioration sensor 139b outputs light amounts of respective colors of RGB received by the RGB sensor 73 as an electric signal to the external device via the connector 78.

The lubricant deterioration sensor 139b may additionally mount another sensor other than the RGB sensor 73. For example, in the lubricant deterioration sensor 139b, when a temperature sensor for detecting the temperature of the lubricant 131a is contained in the electronic component group 70, the temperature detected by the temperature sensor can also be outputted as an electric signal to the external device via the connector 78.

Next, the explanation will be made as to a method of adjusting the direction of the opening 60b of the oil gap 60a of the lubricant deterioration sensor 139b. Hereinafter, although the explanation is made as to the lubricant deterioration sensor 139b, the method of each of the lubricant deterioration sensors such as the lubricant deterioration sensors 137a, 137b, 139a other than the lubricant deterioration sensor 139b is substantially same.

The external device of the lubricant deterioration sensor 139b can specify the kinds and amounts of the contaminant within the lubricant 131a of the reducer 131 based on the colors detected by the RGB sensor 73. That is, the lubricant deterioration sensor 139b can detect the deterioration degree of the lubricant 131a by detecting the colors of the contaminant within the lubricant 131a.

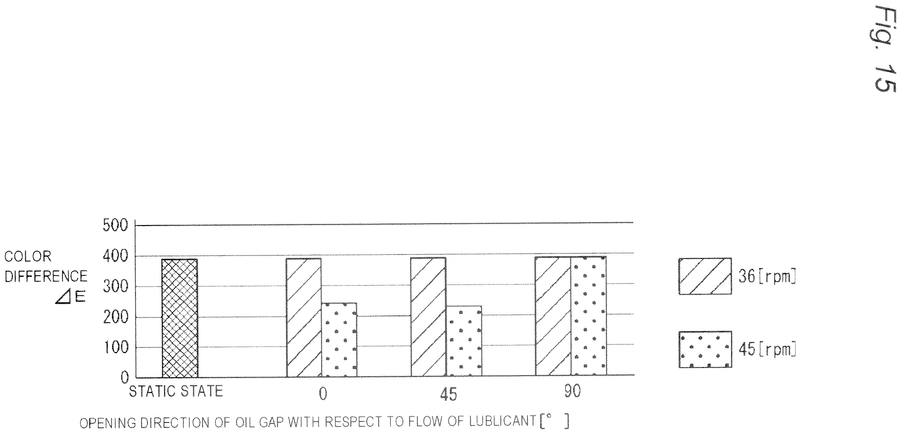

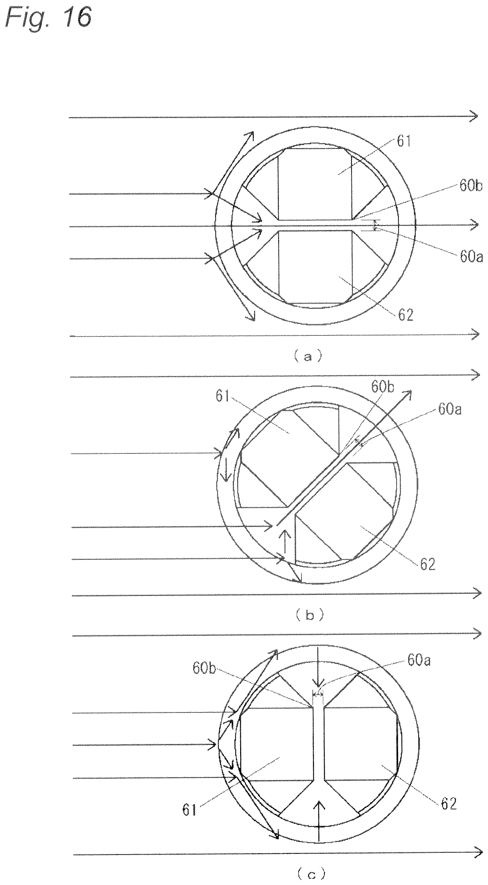

Fig. 15 is a diagram showing an example of the relation between the direction of the opening 60b of the oil gap 60a with respect to the flow of the lubricant 131a and a color difference ΔE of the colors detected by the RGB sensor 73 with respect to black. Fig. 16(a) is a diagram showing a state that the direction of the opening 60b of the oil gap 60a with respect to the flow of the lubricant 131a is 0 degree. Fig. 16(b) is a diagram showing a state that the direction of the opening 60b of the oil gap 60a with respect to the flow of the lubricant 131 a is 45 degrees. Fig. 16(c) is a diagram showing a state that the direction of the opening 60b of the oil gap 60a with respect to the flow of the lubricant 131 a is 90 degrees.

The color difference ΔE of the colors detected by the RGB sensor 73 with respect to black can be calculated by an expression shown by the following Math. 1 by using the respective values of the colors RGB detected by the RGB sensor 73.

[Math. 1]

$$\Delta E = \sqrt{R^2 + G^2 + B^2}$$

In the experiment where the relation shown in Fig. 15 was derived, new oil with a low deterioration degree was used as the lubricant 131a.

Further, in Fig. 15, a "static state" represents a time period where the flow of the lubricant 131a stops. When the flow of the lubricant 131a stops, the direction of the opening 60b of the oil gap 60a with respect to the flow of the lubricant 131a does not influence on the ∆E of the colors detected by the RGB sensor 73 with respect to black. Thus, the ∆E of the colors detected by the RGB sensor 73 with respect to black in the "static state" becomes a determination criterion of the relation between the direction of the opening 60b of the oil gap 60a with respect to the flow of the lubricant 131a and the color difference ∆E of the colors detected by the RGB sensor 73 with respect to black.

Further, in Fig. 15, each of 36 [rpm] and 45 [rpm] represents the rotation speed of the arm 113 with respect to the arm 112 as a revolution number per one minute. Since the lubricant deterioration sensor 139b is attached to the arm 113, this sensor moves within the lubricant 131a by the rotation of the arm 113 with respect to the arm 112. In other words, each of 36 [rpm] and 45 [rpm] indirectly represents the speed of the flow of the lubricant 131a with respect to the lubricant deterioration sensor 139b.

In Fig. 16, arrows other than those representing the oil gap 60a represent the flow of the lubricant 131a.

The detection accuracy of the deterioration of the lubricant 131a can be determined by the color difference ∆E of the colors detected by the RGB sensor 73 with respect to black. In other words, in Fig. 15, the detection accuracy of the deterioration of the lubricant 131a degrades in a case that the rotation speed of the arm 113 with respect to the arm 112 is 45 [rpm] and the direction of the opening 60b of the oil gap 60a with respect to the flow of the lubricant 131a is 0 degree or 45 degrees. In this manner, the detection accuracy of the deterioration of the lubricant 131a degrades depending on the direction of the opening 60b of the oil gap 60a with respect to the flow of the lubricant 131a.

The lubricant deterioration sensor 139b is configured to be adjustable in the direction of the opening 60b of the oil gap 60a.

First, each of the bolts 12 with hexagon holes is loosened by a tool inserted into the tool contact portion 12a so that the supporting member 30 becomes rotatable with respect to the housing 20.

Then, in a state that the rotation of the housing 20 with respect to the arm 113 is prevented by grasping the tool contact portion 22 of the housing 20 by the tool, the supporting member 30 is rotated with respect to the housing 20 by the tool inserted into the tool contact portion 51. The direction of the opening 60b of the oil gap 60a changes in accordance with the rotation of the supporting member 30 with respect to the housing 20.

Lastly, each of the bolts 12 with hexagon holes is fastened by the tool inserted into the tool contact portion 12a so that the rotation of the supporting member 30 becomes impossible with respect to the housing 20.

As explained above, since the RGB sensor 73 detects colors with respect to the light having wavelengths not absorbed by the contaminant within the lubricant 131a at the oil gap 60a among the white light emitted by the white LED 72, each of the lubricant deterioration sensors such as the lubricant deterioration sensor 139b can immediately detect the colors of the contaminant within the lubricant 131a of the reducer 131. That is, each of the lubricant deterioration sensors can immediately specify the kinds and amounts of the contaminant within the lubricant 131a of the reducer 131 based on the colors detected by the RGB sensor 73, by using the external device such as a computer. Each of the lubricant deterioration sensors may be configured that the electronic component group 70 contains an electronic component which specifies the kinds and amounts of the contaminant within the lubricant based on the colors detected by the RGB sensor 73.

Generally, in the industrial robot, accuracy of the locus of the arm etc. largely varies depending on the efficiency of the reducer used at the articular portion. Thus, it is important to suitably exchange the reducer for the industrial robot for new one when the efficiency of the reducer degrades. However, in the case of exchanging the reducer for the industrial robot, it is required to stop the industrial robot provided with this reducer for the industrial robot and a production line installing the industrial robot. Thus, in order to grasp the exchange time of the reducer for the industrial robot, it is very important to suitably predict a failure of the reducer for the industrial robot. In this respect, as described above, each of the lubricant deterioration sensors of the industrial robot 100 can immediately specify the kinds and amounts of the contaminant within the lubricant 131a of the reducer 131 based on the colors detected by the RGB sensor 73, by using the external device such as a computer. Thus, the industrial robot 100 and each of the reducers of the industrial robot 100 can predict an immediate failure.

To the lubricant 131a, there is sometimes added various kinds of additive such as friction reducing agent like organic molybdenum such as MoDTC or MoDTP for reducing the friction of a friction surface, extreme-pressure additive such as SP-based additive for improving extreme-pressure lubricity representing the performance for suppressing the sticking of the friction surface, and dispersing agent such as calcium sulfonate for suppressing the generation or adhesion of sludge. These additives are separated from the lubricant 131a in accordance with the deterioration of the lubricant 131a in such a manner that the additive adheres to, binds with or settles on the metal surface of the industrial robot 100 and the reducer. That is, a reduction amount of the additive within the lubricant 131 a can be used in order to predict a failure of each of the industrial robot 100 and the reducers of the industrial robot 100. Each of the lubricant deterioration sensors can specify, based on the detected colors, not only an amount of ion powder within the lubricant 131a but also a deterioration degree of the base oil and increase of the contaminant such as sludge due to the reduction of various kinds of additive added to the lubricant 131a. Thus, each of the industrial robot 100 and the reducers of the industrial robot 100 can improve the prediction accuracy of a failure as compared with a technique of predicting a failure of the reducer only based on a density of iron powder.

Further, in each of the lubricant deterioration sensors such as the lubricant deterioration sensor 139b, the housing 20 supports the supporting member 30 so as to be rotatable so that the direction of the opening 60b of the oil gap 60a changes when the supporting member 30 rotates. Thus, in a case where the housing 20 is fixed to the industrial robot 100, the direction of the opening 60b of the oil gap 60a at the time of fixing the housing 20 to the industrial robot 100 can be adjusted so that the detection accuracy of the deterioration of the lubricant 131a of the industrial robot 100 becomes high. As a result, each of the industrial robot 100 and the reducers of the industrial robot 100 can predict the failure with a high accuracy.

Further, in each of the lubricant deterioration sensors such as the lubricant deterioration sensor 139b, the supporting member 30 includes the tool contact portion 51, as the portion for receiving the rotational driving force with respect to the housing 20 from the outside by the contact force, at a position not contacting with the lubricant 131a when the housing 20 is fixed to the industrial robot 100. Thus, in each of the lubricant deterioration sensors, after the housing 20 is fixed to the industrial robot 100, the direction of the opening 60b of the oil gap 60a at the time of fixing the housing 20 to the industrial robot 100 can be adjusted so that the detection accuracy of the deterioration of the lubricant 131a of the industrial robot 100 becomes high.

Furthermore, in each of the lubricant deterioration sensors such as the lubricant deterioration sensor 139b, each of the bolts 12 with hexagon holes, configured so as to prevent the rotation of the supporting member 30 with respect to the housing 20 by contacting with both the supporting member 30 and the housing 20, includes the tool contact portion 12a, as the portion for receiving the driving force for contacting with both the supporting member 30 and the housing 20 from the outside by the contact force, at the position not contacting with the lubricant 131a when the housing 20 is fixed to the industrial robot 100. Thus, in each of the lubricant deterioration sensors, after the housing 20 is fixed to the industrial robot 100, the direction of the opening 60b of the oil gap 60a at the time of fixing the housing 20 to the industrial robot 100 can be fixed so that the detection accuracy of the deterioration of the lubricant 131a of the industrial robot 100 becomes high.

Furthermore, in each of the lubricant deterioration sensors, since the light emitting element is the white LED for emitting white light, the sensor can be miniaturized as compared with a configuration where the light emitting element is a lamp other than an LED, for example. Thus, each of the industrial robot 100 and the reducers of the industrial robot 100 can be miniaturized. The light emitting element according to this invention may be one other than the white LED. For example, the light emitting element may be a lamp other than an LED. Further, the light emitting element may be configured to include a red LED or a red lamp other than an LED, a green LED or a green lamp other than an LED and a blue LED or a blue lamp other than an LED, to thereby emit white light by composing light of respective colors emitted from these LEDs or these lamps other than LEDs.

Furthermore, in each of the lubricant deterioration sensors, the gap forming member 60 is provided with the light reflection surfaces 61b, 62b for bending the optical path 10a. Thus, as compared with the configuration where the optical path 10a from the white LED 72 to the RGB sensor 73 is straight, the entire configuration can be miniaturized by disposing the white LED 72 and the RGB sensor 73 close to each other. Furthermore, in each of the lubricant deterioration sensors, the gap forming member 60 has a function of bending the optical path 10a as well as a function of forming the oil gap 60a. Thus, the number of the components can be reduced as compared with a configuration where a member for bending the optical path 10a is separately provided in place of the gap forming member 60. As a result, each of the industrial robot 100 and the reducers of the industrial robot 100 can be miniaturized and also reduce the number of the components.

In particular, each of the lubricant deterioration sensors is configured in a manner that the gap forming member 60 is formed by the two right-angle prisms 61, 62 respectively provided with the light reflection surfaces 61b, 62b each for bending the optical path 10a by 90 degrees, and that the optical path 10a is bent by 180 degrees by the light reflection surfaces 61b, 62b of the two right-angle prisms 61, 62 and the oil gap 60a is formed between the two right-angle prisms 61, 62. Thus, the sensor can be miniaturized with the simple configuration having a small number of the components. As a result, each of the industrial robot 100 and the reducers of the industrial robot 100 can be miniaturized with the simple configuration having the small number of the components.

Furthermore, each of the lubricant deterioration sensors is configured in a manner that the sensor has the holder 40 surrounding at least a part of the optical path 10a and the surface of the holder 40 is treated to the treatment for preventing light reflection. Thus, the RGB sensor 73 can be prevented from receiving unnecessary reflection light. As a result, as compared with a configuration where the RGB sensor 73 receives unnecessary reflection light, each of the lubricant deterioration sensors can improve the detection accuracy of the colors of the contaminant within the lubricant 131a. Accordingly, each of the industrial robot 100 and the reducers of the industrial robot 100 can improve the prediction accuracy of a failure.

Furthermore, in each of the lubricant deterioration sensors, an oil repelling treatment may be performed on the surface planes of the gap forming member 60 forming the oil gap 60a, that is, the light emission surface 61c of the right-angle prism 61 and the light incident surface 62a of the right-angle prism 62. In each of the lubricant deterioration sensors, when each of the light emission surface 61c of the right-angle prism 61 and the light incident surface 62a of the right-angle prism 62 is treated to the oil repelling treatment, the lubricant 131a flows through the oil gap 60a easily. Thus, as compared with a configuration where the lubricant 131a likely remains at the oil gap 60a, the detection accuracy of the colors of the contaminant within the lubricant 131a can be improved. Furthermore, in each of the lubricant deterioration sensors, when each of the light emission surface 61c of the right-angle prism 61 and the light incident surface 62a of the right-angle prism 62 is treated to the oil repelling treatment, dirt is unlikely adhered to each of the light emission surface 61c of the right-angle prism 61 and the light incident surface 62a of the right-angle prism 62. Thus, the degradation of the detection accuracy of the colors of the contaminant within the lubricant 131a due to the adhesion of dirt can be suppressed. As a result, each of the industrial robot 100 and the reducers of the industrial robot 100 can improve the prediction accuracy of a fault.

Although each of the right-angle prisms 61, 62 of the gap forming member 60 is made of glass in this embodiment, each of them may be formed by material such as silicon resin other than glass. When each of the prisms 61, 62 is formed by silicon resin, dirt can be unlikely adhered to the surface planes thereof forming the oil gap 60a.

Although the gap forming member 60 is configured by the two right-angle prisms 61, 62 in this embodiment, the gap forming member may be configured by three or more prisms.

In each of the lubricant deterioration sensors, the white LED 72 and the RGB sensor 73 may be configured in an arrangement other than that explained in this embodiment. For example, in each of the lubricant deterioration sensors, the optical path 10a from the white LED 72 to the RGB sensor 73 may be straight.

Further, in each of the lubricant deterioration sensors, the optical path 10a may be bent by employing the configuration other than the right-angle prisms.

In each of the lubricant deterioration sensors, for example, a battery such as a battery cell may be used as an electric power supply means, and a wireless communication may be employed as a means for outputting the detection result to the external device.

Further, the mounting position of the lubricant deterioration sensor is not limited to that shown in this embodiment, and preferably may be set suitably according to the usage etc. of the industrial robot 100.

INDUSTRIAL APPLICABILITY

According to this invention, the lubricant deterioration sensor is provided which can immediately specify the kinds and amounts of the contaminant within the lubricant of the machine.

REFEERNCE NUMERALS AND SIGNS

| | |
|---|---|
| 10a | optical path |
| 12 | bolt with hexagon hole (rotation preventing member) |
| 12a | tool contact portion (contact driving force receiving portion) |
| 20 | housing (fixing member) |
| 30 | supporting member |
| 40 | holder (optical path surrounding portion) |
| 51 | tool contact portion (rotational driving force receiving portion) |
| 60 | gap forming member |
| 60a | oil gap |
| 60b | opening |
| 61 | right-angle prism |
| 61b | light reflection surface |
| 61c | light emission surface (surface plane forming oil gap) |
| 62 | right-angle prism |
| 62a | light incident surface (surface plane forming oil gap) |

| | |
|---|---|
| 62b | light reflection surface |
| 72 | white LED (light emitting element) |
| 73 | RGB sensor (color light reception element) |
| 100 | industrial robot (machine) |
| 112-116 | arm |
| 120, 130, 140, 150, 160, 170 | articular portion |
| 131 | reducer (reducer for industrial robot) |
| 131a | lubricant |
| 132 | reducer body (machine) |
| 137a, 137b, 139a, 139b | lubricant deterioration sensor |

**Claims**

1. A lubricant deterioration sensor (137a, 137b, 139a, 139b) to be fixed to a machine to detect deterioration of a lubricant (131a) in a lubricant-containing space of the machine, the lubricant deterioration sensor (137a, 137b, 139a, 139b) comprising:

   a light emitting element (72) configured to emit light;
   a color light reception element (73) configured to receive the light emitted from the light emitting element (72) and detect color of the received light;
   a gap forming member (60) forming an oil gap (60a) being a gap in which the lubricant (131a) enters;
   a supporting member (30) supporting the light emitting element (72), the color light reception element (73) and the gap forming member (60); and
   a fixing member (20) to be fixed to the machine, wherein
   the gap forming member (30) is disposed such that, when the lubricant deterioration sensor (137a, 137b, 139a, 139b) is fixed to the machine, the gap forming member (60) is in the lubricant-containing space of the machine such that lubricant enters the oil gap (60a),
   the gap forming member (60) transmits the light emitted from the light emitting element (72), and
   the oil gap (60a) is disposed on an optical path (10a) from the light emitting element (72) to the color light reception element (73);
   **characterized in that**
   the fixing member (20) supports the supporting member (30) rotatably so that a direction of an opening (60b) of the oil gap (60a) changes when the supporting member (30) rotates relative to the fixing member (20).

2. The lubricant deterioration sensor (137a, 137b, 139a, 139b) according to claim 1, wherein
   the supporting member (30) includes a rotational driving force receiving portion (51) which is a portion for receiving a rotational driving force with respect to the supporting member (30) from outside by a contact force, at a position where the rotational driving force receiving portion (51) does not contact with the lubricant (131a) in a state where the fixing member (20) is fixed to the machine.

3. The lubricant deterioration sensor (137a, 137b, 139a, 139b) according to claim 1 or 2, further comprising:

   a rotation preventing member (12) preventing a rotation of the supporting member (30) with respect to the fixing member (20) by contacting with both the supporting member (30) and the fixing member (20), wherein
   the rotation preventing member (12) includes a contact driving force receiving portion (12a) which is a portion for receiving a driving force for contacting with both the supporting member (30) and the fixing member (20) from outside by a contact force, at a position where the contact driving force receiving portion (12a) does not contact with the lubricant (131a) in a state where the fixing member (20) is fixed to the machine.

4. The lubricant deterioration sensor (137a, 137b, 139a, 139b) according to any one of claims 1 to 3, wherein
   the light emitting element (72) is a white LED which emits white light.

5. The lubricant deterioration sensor (137a, 137b, 139a, 139b) according to any one of claims 1 to 4, wherein
   the gap forming member (60) has a reflection surface (61b, 62b) for bending the optical path (10a).

6. The lubricant deterioration sensor (137a, 137b, 139a, 139b) according to claim 5, wherein
   the gap forming member (60) includes two right-angle prisms (61, 62) each provided with the reflection surface (61b, 62b) for bending the optical path (10a) by 90 degrees, the optical path (10a) being bent by 180 degrees by the reflection surfaces (61b, 62b) of the two right-angle prisms (61, 62), and
the oil gap (60a) is formed between the two right-angle prisms (61, 62).

7. The lubricant deterioration sensor (137a, 137b, 139a, 139b) according to any one of claims 1 to 6, wherein
the supporting member (30) has an optical path surrounding portion (40) which surrounds at least a part of the optical path (10a), and
the optical path surrounding portion (40) has a surface which is treated for preventing light reflection.

8. The lubricant deterioration sensor (137a, 137b, 139a, 139b) according to any one of claims 1 to 7, wherein
a surface of the gap forming member (60) forming the oil gap (60a) is treated by an oil repelling treatment.

9. A reduction gear (131) for an industrial robot (100), comprising:
the lubricant deterioration sensor (137a, 137b, 139a, 139b) described in any one of claims 1 to 8; and a reduction gear body (132) as the machine.

10. An industrial robot (100), comprising:

the lubricant deterioration sensor (137a, 137b, 139a, 139b) described in any one of claims 1 to 8; and
an industrial robot (100) body as the machine, wherein
the industrial robot (100) body includes an arm (112, 113, 114, 115, 116) and a reduction gear (131) used at an articular portion (120, 130, 140, 150, 160, 170) of the arm (112 - 116), and the lubricant deterioration sensor (137a, 137b, 139a, 139b) is a sensor for detecting deterioration of lubricant (131a) of the reduction gear (131).

## Patentansprüche

1. Ein Schmiermittelzersetzungssensor (137a, 137b, 139a, 139b), der an einer Maschine zu befestigen ist, um Zersetzung eines Schmiermittels (131a) in einem schmiermittelenthaltenden Raum der Maschine zu detektieren, wobei der Schmiermittelzersetzungssensor (137a, 137b, 139a, 139b) umfasst:

ein lichtemittierendes Element (72), das konfiguriert ist, Licht zu emittieren;
ein Farblichtempfangselement (73), das konfiguriert ist, das von dem lichtemittierenden Element (72) emittierte Licht zu empfangen und Farbe des empfangenen Lichts zu detektieren; ein spaltbildendes Element (60), das einen Ölspalt (60a) ausbildet, der ein Spalt ist, in den das Schmiermittel (131a) eindringt;
ein Stützelement (30), das das lichtemittierende Element (72), das Farblichtempfangselement (73) und das spaltbildende Element (60) stützt; und
ein Befestigungselement (20), das an der Maschine zu befestigen ist, wobei das spaltbildende Element (30) so angeordnet ist, dass, wenn der Schmiermittelzersetzungssensor (137a, 137b, 139a, 139b) an der Maschine befestigt ist, sich das spaltbildende Element (60) in dem schmiermittelenthaltenden Raum so befindet, dass Schmiermittel in den Ölspalt (60a) eindringt,
das spaltbildende Element (60) das von dem lichtemittierenden Element (72) emittierte Licht überträgt, und
der Ölspalt (60a) an einem optischen Pfad (10a) von dem lichtemittierenden Element (72) zu dem Farblichtempfangselement (73) angeordnet ist;
**dadurch gekennzeichnet, dass**
das Befestigungselement (20) das Stützelement (30) rotierbar stützt, sodass sich eine Richtung einer Öffnung (60b) des Ölspalts (60a) ändert, wenn das Stützelement (30) relativ zu dem Befestigungselement (20) rotiert.

2. Der Schmiermittelzersetzungssensor (137a, 137b, 139a, 139b) nach Anspruch 1, wobei das Stützelement (30) einen Rotationsantriebskraftempfangsabschnitt (51), der ein Abschnitt zum Empfangen einer Rotationsantriebskraft bezüglich des Stützelements (30) von außerhalb durch eine Kontaktkraft ist, an einer Position beinhaltet, an der der Rotationsantriebskraftempfangsabschnitt (51) nicht mit dem Schmiermittel (131a) in einem Zustand kontaktiert, in dem das Befestigungselement (20) an der Maschine befestigt ist.

3. Der Schmiermittelzersetzungssensor (137a, 137b, 139a, 139b) nach Anspruch 1 oder 2, weiterhin umfassend:

ein Rotationsverhinderungselement (12), das eine Rotation des Stützelements (30) bezüglich des Befestigungselements (20) durch Kontaktieren mit beiden von dem Stützelement (30) und dem Befestigungselement (20) verhindert, wobei

das Rotationsverhinderungselement (12) einen Kontaktantriebskraftempfangsabschnitt (12a), der ein Abschnitt zum Empfangen einer Antriebskraft zum Kontaktieren mit beiden von dem Stützelement (30) und dem Befestigungselement (20) von außerhalb durch eine Kontaktkraft ist, an einer Position beinhaltet, an der der Kontaktantriebskraftempfangsabschnitt (12a) nicht mit dem Schmiermittel (131a) in einem Zustand kontaktiert, in dem das Befestigungselement (20) an der Maschine befestigt ist.

4. Der Schmiermittelzersetzungssensor (137a, 137b, 139a, 139b) nach irgendeinem der Ansprüche 1 bis 3, wobei das lichtemittierende Element (72) eine weiße LED ist, die weißes Licht emittiert.

5. Der Schmiermittelzersetzungssensor (137a, 137b, 139a, 139b) nach irgendeinem der Ansprüche 1 bis 4, wobei das spaltbildende Element (60) eine Reflexionsfläche (61b, 62b) zum Biegen des optischen Pfads (10a) aufweist.

6. Der Schmiermittelzersetzungssensor (137a, 137b, 139a, 139b) nach Anspruch 5, wobei das spaltbildende Element (60) zwei rechtwinklige Prismen (61, 62) beinhaltet, die jeweils mit der Reflexionsfläche (61b, 62b) zum Biegen des optischen Pfads (10a) um 90 Grad versehen sind, wobei der optische Pfad (10a) um 180 Grad durch die Reflexionsflächen (61b, 62b) der zwei rechtwinkligen Prismen (61, 62) gebogen wird, und der Ölspalt (60a) zwischen den zwei rechtwinkligen Prismen (61, 62) ausgebildet ist.

7. Der Schmiermittelzersetzungssensor (137a, 137b, 139a, 139b) nach irgendeinem der Ansprüche 1 bis 6, wobei das Stützelement (30) einen Umgebungsabschnitt des optischen Pfads (40) aufweist, der zumindest einen Teil des optischen Pfads (10a) umgibt, und der Umgebungsabschnitt des optischen Pfads (40) eine Oberfläche aufweist, die zum Verhindern von Lichtreflexion behandelt ist.

8. Der Schmiermittelzersetzungssensor (137a, 137b, 139a, 139b) nach irgendeinem der Ansprüche 1 bis 7, wobei eine Oberfläche des spaltbildenden Elements (60), das den Ölspalt (60a) bildet, durch eine ölabweisende Behandlung behandelt wird.

9. Ein Untersetzungsgetriebe (131) für einen Industrieroboter (100), umfassend:

den Schmiermittelzersetzungssensor (137a, 137b, 139a, 139b), der in irgendeinem der Ansprüche 1 bis 8 beschrieben ist; und
einen Untersetzungsgetriebekörper (132) als die Maschine.

10. Einen Industrieroboter (100), umfassend:

den Schmiermittelzersetzungssensor (137a, 137b, 139a, 139b), der in irgendeinem der Ansprüche 1 bis 8 beschrieben ist; und
einen Industrieroboter- (100)-Körper als die Maschine, wobei
der Industrieroboter- (100)-Körper einen Arm (112, 113, 114, 115, 116) und ein Untersetzungsgetriebe (131) beinhaltet, das bei einem Gelenkabschnitt (120, 130, 140, 150, 160, 170) des Arms (112 - 116) verwendet wird, und
der Schmiermittelzersetzungssensor (137a, 137b, 139a, 139b) ein Sensor zum Detektieren von Zersetzung von Schmiermittel (131a) des Untersetzungsgetriebes (131) ist.

**Revendications**

1. Capteur de dégradation de lubrifiant (137a, 137b, 139a, 139b) à fixer sur une machine pour détecter la dégradation d'un lubrifiant (131a) dans un espace de la machine contenant du lubrifiant, le capteur de dégradation de lubrifiant (137a, 137b, 139a, 139b) comprenant:

un élément d'émission de lumière (72) configuré pour émettre de la lumière;
un élément de réception de lumière colorée (73) configuré pour recevoir la lumière émise par l'élément d'émission de lumière (72) et pour détecter la couleur de la lumière reçue;
un élément de formation d'espace (60) formant un espace d'huile (60a) qui est un espace dans lequel pénètre le lubrifiant (131a);

un élément de support (30) supportant l'élément d'émission de lumière (72), l'élément de réception de lumière colorée (73) et l'élément de formation d'espace (60); et

un élément de fixation (20) à fixer sur la machine, dans lequel l'élément de formation d'espace (30) est disposé de telle sorte que, lorsque le capteur de dégradation de lubrifiant (137a, 137b, 139a, 139b) est fixé à la machine, l'élément de formation d'espace (60) se trouve dans l'espace contenant le lubrifiant de la machine de telle sorte que le lubrifiant entre dans l'espace d'huile (60a),

l'élément de formation d'espace (60) transmet la lumière émise par l'élément d'émission de lumière (72), et

l'espace d'huile (60a) est disposé sur un chemin optique (10a) allant de l'élément émetteur de lumière (72) à l'élément récepteur de lumière colorée (73);

**caractérisé en ce que**

l'élément de fixation (20) supporte l'élément de support (30) de manière rotative de sorte qu'une direction d'une ouverture (60b) de l'espace d'huile (60a) change lorsque l'élément de support (30) tourne par rapport à l'élément de fixation (20).

2. Capteur de dégradation de lubrifiant (137a, 137b, 139a, 139b) selon la revendication 1, dans lequel l'élément de support (30) comprend une partie de réception de force d'entraînement en rotation (51) qui est une partie pour recevoir une force d'entraînement en rotation par rapport à l'élément de support (30) depuis l'extérieur par une force de contact, à une position où la partie de réception de force d'entraînement en rotation (51) n'est pas en contact avec le lubrifiant (131a) dans un état où l'élément de fixation (20) est fixé à la machine.

3. Capteur de dégradation de lubrifiant (137a, 137b, 139a, 139b) selon les revendications 1 ou 2, comprenant en outre:

un élément de prévention de rotation (12) empêchant une rotation de l'élément de support (30) par rapport à l'élément de fixation (20) en entrant en contact à la fois avec l'élément de support (30) et l'élément de fixation (20), dans lequel

l'élément de prévention de rotation (12) comprend une partie de réception de force d'entraînement de contact (12a) qui est une partie destinée à recevoir une force d'entraînement pour entrer en contact avec à la fois l'élément de support (30) et l'élément de fixation (20) depuis l'extérieur par une force de contact, à une position où la partie de réception de force d'entraînement de contact (12a) n'entre pas en contact avec le lubrifiant (131a) dans un état où l'élément de fixation (20) est fixé à la machine.

4. Capteur de dégradation de lubrifiant (137a, 137b, 139a, 139b) selon l'une quelconque des revendications 1 à 3, dans lequel l'élément émetteur de lumière (72) est une DEL blanche qui émet une lumière blanche.

5. Capteur de dégradation de lubrifiant (137a, 137b, 139a, 139b) selon l'une quelconque des revendications 1 à 4, dans lequel l'élément de formation d'espace (60) a une surface de réflexion (61b, 62b) pour courber le chemin optique (10a).

6. Capteur de dégradation de lubrifiant (137a, 137b, 139a, 139b) selon la revendication 5, dans lequel l'élément de formation d'espace (60) comprend deux prismes à angle droit (61, 62), chacun étant pourvu de la surface de réflexion (61b, 62b) pour courber le chemin optique (10a) de 90 degrés, le chemin optique (10a) étant courbé de 180 degrés par les surfaces de réflexion (61b, 62b) des deux prismes à angle droit (61, 62), et l'espace d'huile (60a) est formé entre les deux prismes à angle droit (61, 62).

7. Capteur de dégradation de lubrifiant (137a, 137b, 139a, 139b) selon l'une quelconque des revendications 1 à 6, dans lequel l'élément de support (30) a une partie d'entourage de chemin optique (40) qui entoure au moins une partie du chemin optique (10a), et la partie entourant le chemin optique (40) a une surface qui est traitée pour empêcher la réflexion de la lumière.

8. Capteur de dégradation de lubrifiant (137a, 137b, 139a, 139b) selon l'une quelconque des revendications 1 à 7, dans lequel une surface de l'élément de formation d'espace (60) formant l'espace d'huile (60a) est traitée par un traitement oléofuge.

9. Réducteur (131) pour un robot industriel (100), comprenant:

le capteur de dégradation de lubrifiant (137a, 137b, 139a, 139b) décrit dans l'une quelconque des revendications 1 à 8 ; et

un corps de réducteur (132) en tant que machine.

10. Robot industriel (100), comprenant:

le capteur de dégradation de lubrifiant (137a, 137b, 139a, 139b) décrit dans l'une quelconque des revendications 1 à 8; et

un corps de robot industriel (100) comme machine, dans lequel

le corps du robot industriel (100) comprend un bras (112, 113, 114, 115, 116) et un réducteur (131) utilisé au niveau d'une partie articulaire (120, 130, 140, 150, 160, 170) du bras (112 - 116), et

le capteur de dégradation de lubrifiant (137a, 137b, 139a, 139b) est un capteur pour détecter la dégradation du lubrifiant (131a) du réducteur (131).

Fig. 1

Fig. 2

*Fig. 3*

139b

12    12a

22

14

20
21

30

60a    60b

# Fig. 4

Fig. 5

( a )

( b )

Fig. 6

(a)

(b)

# Fig. 7

（a）

（b）

*Fig. 8*

( a )

( b )

*Fig. 9*

40

(a)

40

48
48
47
47
44
43
46
45
49
42
41
42a
41a

(b)

*Fig. 10*

40

41a

(a)

48 — 48

47 — 47

49

42

42a

(b)

*Fig. 11*

( a )

( b )

Fig. 12

*Fig. 13*

50

( a )

50

52   51

53   53

( b )

Fig. 14

(a)

(b)

## Fig. 15

Fig. 16

(a)

(b)

(c)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5296843 A
- US 2009216464 A1
- US 2008024761 A1
- EP 2647979 A1
- JP 7146233 A
- JP 10104160 A

### Non-patent literature cited in the description

- METHOD OF DISCRIMINATING HUE OF CONTAMINANT WITHIN LUBRICANT. **TOMOHIKO YAMAGUCHI.** Faculty of Engineering, Research Report. Fukui University, March 2003, vol. 51, 81-88
- **TOMONORI HONDA.** DETERIORATION DIAGNOSIS OF LUBRICANT INSPECTION TECHNOLOGY. *Journal of Japan Society for Precision Engineering,* 2009, vol. 75 (3), 359-362